# FASCICULE DE BREVET EUROPEEN

(11) **EP 3 727 662 B1**
(45) Date de publication et mention de la délivrance du brevet: **26.01.2022**
(21) Numéro de dépôt: 18842800.7
(22) Date de dépôt: 21.12.2018
(51) Int. Cl.: C08F 290/04, B01F 17/00

(54) **ÉMUSLION SUBMICRONIQUE**
SUBMIKRONISCHE EMULSION
SUBMICRONIC EMULSION

(30) Priorité: 21.12.2017 FR 1762696
(43) Date de publication de la demande: 28.10.2020
(73) Titulaire: Coatex, 69730 Genay (FR)
(72) Inventeur: CHAMPAGNE, Clémentine, 69300 CALUIRE-ET-CUIRE (FR); SUAU, Jean-Marc, 69480 LUCENAY (FR)
(74) Mandataire: Balmefrezol, Ludovic Francis Pierre
(86) Numéro de dépôt international: PCT/FR2018/053516
(87) Numéro de publication internationale: WO 2019/122784

(56) Documents cités:
- EP-A1- 1 172 077
- WO-A1-2008/087211
- WO-A1-2017/182265
- WO-A2-2015/155703
- US-A1- 2007 036 831

## Description

L'invention concerne une méthode de préparation d'une dispersion de composé lipophile dans une phase continue hydrophile dont la morphologie est submicronique. Lors de la préparation de cette dispersion, on applique, au moyen d'un dispositif produisant un gradient de cisaillement inférieur à 5 000 s⁻¹, une contrainte de cisaillement allant de 100 à 5 000 Pa lors de l'addition du composé lipophile dans la phase continue qui comprend un composé modificateur de rhéologie choisi parmi les copolymères anioniques, de préférence un polymère anionique, en particulier un polymère ASE ou un polymère HASE. Les particules de composé lipophile dispersées sont des particules nanométriques dont la taille est inférieure à 1 µm. La dispersion, notamment sous la forme d'une émulsion, peut être mise en œuvre dans de nombreux domaines.

De nombreux domaines requièrent la combinaison de substances possédant des propriétés qui les rendent incompatibles. En particulier, il est important de pouvoir associer un composé lipophile et un composé hydrophile.

Ainsi, il est essentiel de pouvoir disposer de méthodes de préparation de dispersion d'un composé lipophile dans une phase hydrophile.

Outre le fait de pouvoir préparer de telles dispersions, notamment sous la forme d'émulsions, il est également essentiel de fournir des méthodes conduisant à des dispersions stables.

L'émulsification de deux substances incompatibles est un procédé hors d'équilibre qui consiste à rompre des domaines macroscopiques de la substance à disperser par apport d'énergie mécanique et à stabiliser la dispersion obtenue.

Généralement, les méthodes de préparation de dispersion, en particulier d'émulsion, mettent en œuvre une contrainte physique lors du mélange des deux phases à combiner. Appliquer une contrainte de cisaillement lors de cette combinaison est un mode opératoire très répandu. La valeur de la contrainte de cisaillement qui est appliquée est un facteur essentiel du contrôle de la taille des particules de composé lipophile qui sont dispersées dans la phase hydrophile.

Le plus souvent, la phase hydrophile est sous une forme gélifiée, notamment pour faciliter la préparation de la dispersion ou pour améliorer la stabilité de cette dispersion.

Un inconvénient majeur rencontré lors de la préparation des dispersions concerne la déstabilisation, voire la rupture, du gel de la phase hydrophile, en particulier cette déstabilisation ou cette rupture lors de l'application de la contrainte de cisaillement. Lorsqu'elle se produit, cette déstabilisation ou cette rupture empêche d'atteindre des tailles de particules submicrométriques ou nanométriques.

Les dispersions alors obtenues ne sont pas conformes au but recherché et elles sont alors généralement inutilisables.

En effet, de nombreux domaines techniques nécessitent la mise en œuvre de dispersions dans lesquelles le composé lipophile est sous la forme de particules de très petite taille et en particulier de taille submicronique. Il est donc essentiel de disposer de méthodes de préparation de dispersion de composé lipophile sous la forme de particules nanométriques. Par ailleurs, il est également essentiel de pouvoir préparer des dispersions en utilisant des dispositifs mécaniques usuels, notamment des mixeurs ou des mélangeurs largement disponibles. En particulier, il est particulièrement avantageux de pouvoir préparer des dispersions en employant des mixeurs ou des mélangeurs produisant un gradient de cisaillement peu élevé, par exemple inférieur à 5 000 s⁻¹, voire inférieur à 2 000 s⁻¹ ou moins.

De même, il est essentiel de pouvoir disposer de méthodes de préparation de dispersion au cours desquelles la phase continue hydrophile est stable, en particulier est sous la forme d'un gel stable et n'est pas rompu ou fracturé lors de l'application d'une contrainte mécanique.

De manière préférée, les méthodes de préparation de dispersion devraient permettre de contrôler la texture des dispersions préparées.

Le document WO 2015 155703 décrit une nanodispersion comprenant un milieu de dispersion aqueux, une phase dispersée, un agent tensio-actif et éventuellement un additif. Cette nanodispersion est auto-émulsifiante et ne comprend aucun copolymère anionique. Le document EP 1951200 divulgue une composition comprenant une nanoémulsion utilisable pour son activité anti-inflammatoire. Le document WO 2017 182265 divulgue une nanoémulsion comprenant une quantité importante de composé tensio-actif qui est un un sel N-acylé d'un amino-monocarboxylique. Le document EP 1172077 décrit une nanoémulsion huile-dans-eau dont les gouttelettes d'huile sont constituées d'un lipide amphiphile non-ionique ou anionique. Elle ne comprend aucun copolymère anionique. Les méthodes de préparation de dispersion de l'état de la technique ne permettent pas de préparer des dispersions de manière satisfaisante ou efficace.

La méthode selon l'invention permet d'apporter une solution à tout ou partie des méthodes de préparation de dispersion de l'état de la technique.

Ainsi, l'invention fournit une méthode de préparation d'une dispersion (D) comprenant :
- une phase continue hydrophile comprenant au moins un composé hydrophile et au moins un composé modificateur de rhéologie du composé hydrophile, et choisi parmi les copolymères anioniques, et
- une phase lipophile dispersée dans la phase continue sous la forme de particules nanométriques,
comprenant :
- la préparation d'un mélange (M) comprenant le composé hydrophile et le composé modificateur de rhéologie du composé hydrophile,
- l'addition du composé lipophile dans la phase continue en appliquant, au moyen d'un dispositif produisant un gradient de cisaillement inférieur à 5 000 s⁻¹, une contrainte choisie parmi une contrainte de cisaillement allant de 100 à 5 000 Pa et une contrainte élongationnelle allant de 100 à 5 000 Pa.

De manière préférée, la dispersion (D) est une émulsion. De manière plus préférée, la dispersion est une émulsion de la phase dispersée lipophile dans la phase continue hydrophile.

De manière particulièrement avantageuse, la méthode selon l'invention permet de préparer la dispersion (D) en l'absence de composé tensio-actif qui sert généralement à provoquer l'auto-émulsion de la phase lipophile à disperser.

La méthode selon l'invention permet de préparer aisément une dispersion (D) dans laquelle la phase lipophile est dispersée dans la phase continue hydrophile sous la forme de particules nanométriques. Selon l'invention, les particules de phase lipophile ont donc une taille inférieure à 1 µm, donc strictement inférieure à 1 000 nm. La méthode selon l'invention permet donc de préparer une dispersion (D) pour laquelle les particules de phase lipophile dispersée ont une taille moyenne (mesurée par diffraction de la lumière) submicronique.

De manière préférée, au moins 30 % en volume ou au moins 40 % en volume, plus préférentiellement au moins 50 % en volume, des particules de phase lipophile dispersées ont une taille moyenne nanométrique ou submicronique.

De manière plus préférée, au moins 30 % en volume ou au moins 40 % en volume, plus préférentiellement au moins 50 % en volume, des particules de phase lipophile dispersées ont une taille moyenne allant de 50 à 999 nm ou de 100 à 999 nm ou encore de 50 à 990 nm ou de 500 à 990 nm.

La méthode selon l'invention permet de contrôler la viscosité de la phase continue hydrophile préparée. Selon l'invention, la viscosité est mesurée au moyen d'un rhéomètre, par exemple au moyen d'un rhéomètre Mars III (Thermofisher).

De manière préférée selon l'invention, la phase continue hydrophile a une viscosité allant de 20 à 50 000 mPa.s. De manière plus préférée selon l'invention, la phase continue hydrophile a une viscosité allant de 100 à 50 000 mPa.s ou de 100 à 20 000 mPa.s.

La méthode selon l'invention permet également de contrôler la viscosité de la dispersion (D) préparée. De manière préférée selon l'invention, la dispersion (D) a une viscosité allant de 20 à 50 000 mPa.s. De manière plus préférée selon l'invention, la dispersion (D) a une viscosité allant de 100 à 50 000 mPa.s ou de 100 à 20 000 mPa.s. Selon l'invention, la préparation de la dispersion (D) peut comprendre une étape supplémentaire permettant d'augmenter sa viscosité. Par ailleurs, une étape supplémentaire de réduction du pH peut permettre d'abaisser la viscosité de la dispersion (D).

La méthode de préparation selon l'invention permet de mettre en œuvre des composés lipophiles très variés. De manière préférée, le composé lipophile mis en œuvre selon l'invention est un composé lipophile utile dans un domaine choisi parmi la cosmétique, la peinture, la teinture, l'imprimerie, les encres, la construction, les combustibles, les lubrifiants, les agents anti-mousse, la métallurgie, les fertilisants, la pharmacie, l'agrochimie, les produits phytosanitaires, la détergence, l'alimentation, le cuir, l'enduction, notamment l'enduction textile.

De manière plus préférée selon l'invention, le composé lipophile est un composé non-miscible dans l'eau à température ambiante ou bien miscible dans l'eau à température ambiante en une quantité en poids inférieure à 0,1 % en poids par rapport à la quantité d'eau.

De manière particulièrement avantageuse, au sein de la dispersion (D) les quantités respectives de phases hydrophile et lipophile peuvent varier assez largement. De préférence, la dispersion (D) comprend de 0,1 à 75 % en poids ou de 0,3 à 75 % en poids ou de 1 à 75 % en poids de phase lipophile dispersée par rapport à la quantité totale en poids de phase hydrophile continue et de phase lipophile dispersée.

Également de préférence, la dispersion (D) comprend de 0,1 à 70 % en poids ou de 0,3 à 70 % en poids ou de 1 à 70 % en poids de phase lipophile dispersée par rapport à la quantité totale en poids de phase hydrophile continue et de phase lipophile dispersée.

Plus préférentiellement la dispersion (D) comprend de 0,1 à 65 % en poids ou de 0,3 à 65 % en poids ou de 1 à 65 % en poids de phase lipophile dispersée par rapport à la quantité totale en poids de phase hydrophile continue et de phase lipophile dispersée.

Également plus préférentiellement, la dispersion (D) comprend de 0,1 à 60 % en poids ou de 0,3 à 60 % en poids ou de 1 à 60 % en poids, de phase lipophile dispersée par rapport à la quantité totale en poids de phase hydrophile continue et de phase lipophile dispersée. De manière préférée selon l'invention, le composé hydrophile est l'eau. Le composé hydrophile peut également être de l'eau en mélange avec au moins un autre composé. De préférence, ce mélange comprend de l'eau et un composé choisi parmi glycérol, polyglycérols, glycols, par exemple propylèneglycol, butylèneglycol, composés humectants, par exemple des composés humectants pour composition cosmétique, dérivés de sucres, par exemple xylytol, maltilol, agents de coalescence, par exemple des polyalkylene-glycols de faible masse moléculaire, notamment polyéthylèneglycol, butyldiglycol.

Selon l'invention, les composés humectants préférés sont hydrosolubles ou hydrophiles. De manière préférée, ils sont choisis parmi un composé hydrosoluble d'origine synthétique, un composé hydrosoluble d'origine naturelle et leurs combinaisons, en particulier un composé hydrosoluble d'origine végétale ; par exemple un composé hydrosoluble (a) choisi parmi diols, triols, sucres, sucres modifiés, éthers, composés protéiques, acides aminés, triglycérides et leurs combinaisons ; notamment un composé hydrosoluble (a) choisi parmi acide pidolique (PCA ; numéro CAS 98-79-3 forme L ou S(-) ; 4042-36-8 forme D ou R(+) ; 149-87-1 forme racémique) ; dérivés de PCA, notamment arginine PCA, chitosan PCA, dérivé de cuivre de PCA, éthylhexyl PCA, lauryl PCA, dérivé de magnésium de PCA, dérivé de sodium de PCA, dérivé de zinc de PCA ; butylène glycol, pentylène glycol ; gluconate de calcium ; fructose ; glucose ; isomalt ; lactose ; maltitol ; mannitol ; polydextrose ; sorbitol ; saccharose ; sucrose ; xylitol ; glycérol ; glycérine ; acide glycyrrhizique ; dérivés d'acide glycyrrhizique ; histidine ; acide hyaluronique ; sels d'acide hyaluronique, notamment sodium hyaluronate ; hydrolysat de soie ; hydrolysat de kératine ; hydrolysat de soja ; PEG -7 ; PEG -8 ; PEG -10 ; PEG -12 ; PEG -14 ; phytantriol ; propylène glycol ; soie (*serica*) ; urée ; propylène glycol-1,2,6 ; hexanetriol ; butylène glycol ; capryl glycol ; dipropylène glycol ; erythritol ; triéthylène glycol ; hexylène glycol ; phytantriol hexanediol ; triol de cire d'abeille ; humectants d'origine biologique ; panthenol ; provitamine B5 ; inositol glycogen ; sucres et sucres modifiés polyglyceryl ; sorbitol ; miel ; polyols polymériques ; inositol ; vitamine B7 ; saponine de réglisse à haut pouvoir sucrant ; éthers ; isoceteth-x ; isolaureth-x ; laneth-x ; laureth-x ; steareth-x ; polyethyleneglycols ; dérivés de polyéthylèneglycols ; trideceth-(5-50) ; éther polyéthylène glycol d'alcool tridecylique ; copolyols siliconés ; composés humectants protéiques ; cocodimonium hydroxypropyl de caséine hydrolysée ; cocodimonium hydroxypropyl de collagen hydrolysé ; cocodimonium hydroxypropyl de keratin hydrolysée ; cocodimonium hydroxypropyl de protéines de riz hydrolysées ; cocodimonium hydroxypropyl de protéines de soie hydrolysées ; cocodimonium hydroxypropyl de protéines de soja hydrolysées ; cocodimonium hydroxypropyl de protéines de blé hydrolysées ; cocodimonium hydroxypropyl d'amino-acides de soie ; cocoyl de collagen hydrolysé ; cocoyl de kératine hydrolysé ; kératine ; kératine hydrolysée ; protéines d'avoine hydrolysées ; de protéines quinoa hydrolysées ; potassium cocoyl de collagen hydrolysé ; triéthanolamine-cocoyl hydrolysé de collagen; triéthanolamine-cocoyl de protéines de soja hydrolysées ; histidine ; acides aminés ; triglycérides ; triacétate de glycéryle ; triacétate de glycéryle obtenu par estérification de la glycérine naturelle ; acides alpha-hydroxyle ; dérivés de sucre de fruit ; dérivés de sucre de fruit du lait ; acides de fruit ; acide lactique ; neoagarobiose ; *Aloe vera.*

De manière essentielle, la méthode selon l'invention met en œuvre au moins un composé modificateur de rhéologie du composé hydrophile choisi parmi les copolymères anioniques. Selon l'invention, la concentration du composé modificateur de rhéologie dans le mélange (M) comprenant également le composé hydrophile peut varier assez largement, notamment pour permettre un contrôle efficace de la viscosité du mélange (M).

De manière préférée, la concentration en poids de composé modificateur de rhéologie dans le mélange (M) va de 4 à 14 % en poids de mélange (M) et la viscosité du mélange (M) va de 300 à 5 000 mPa.s.

De manière également préférée, la concentration en poids de composé modificateur de rhéologie dans le mélange (M) va de 4 à 12 % en poids de mélange (M) et la viscosité du mélange (M) va de 300 à 2 000 mPa.s.

De manière également préférée, la concentration en poids de composé modificateur de rhéologie dans le mélange (M) va de 4 à 11 % en poids de mélange (M) et la viscosité du mélange (M) va de 300 à 1 000 mPa.s.

De manière également préférée, la concentration en poids de composé modificateur de rhéologie dans le mélange (M) va de 2,5 à 12 % en poids de mélange (M) et la viscosité du mélange (M) va de 100 à 2 000 mPa.s.

De manière également préférée, la concentration en poids de composé modificateur de rhéologie dans le mélange (M) va de 2,5 à 11 % en poids de mélange (M) et la viscosité du mélange (M) va de 100 à 1 000 mPa.s.

Selon l'invention, le composé modificateur de rhéologie a généralement un pH supérieur à 5, de préférence supérieur à 5,5. Plus préférentiellement le pH est supérieur à 6.

Selon l'invention, le composé modificateur de rhéologie est choisi parmi les copolymères ASE, les copolymères HASE et leurs combinaisons.

Selon l'invention, les copolymères anioniques préférés sont préparés par réaction de polymérisation :
(a1) d'au moins un monomère anionique comprenant au moins une insaturation oléfinique polymérisable, de préférence un monomère anionique comprenant au moins une insaturation oléfinique polymérisable et au moins une fonction acide carboxylique, de préférence le monomère anionique est choisi parmi l'acide acrylique, l'acide méthacrylique, l'acide maléique, l'anhydride maléique, l'acide itaconique, l'acide crotonique, un sel d'acide acrylique, un sel d'acide méthacrylique, un sel d'acide maléique, un sel d'anhydride maléique, un sel d'acide itaconique, un sel d'acide crotonique et leurs combinaisons, bien plus préférentiellement l'acide acrylique ou l'acide méthacrylique et
(a2) d'au moins un ester d'un composé dérivé d'un acide choisi parmi l'acide acrylique, l'acide méthacrylique, l'acide maléique, l'anhydride maléique, l'acide itaconique et l'acide crotonique, de préférence un ester de l'acide acrylique ou un ester de l'acide méthacrylique, de préférence choisi parmi acrylate de méthyle, acrylate d'éthyle, acrylate de propyle, acrylate de butyle, acrylate d'éthylhexyle, méthacrylate de méthyle, méthacrylate d'éthyle, méthacrylate de propyle, méthacrylate de butyle, méthacrylate d'éthylhexyle, et leurs combinaisons.

De manière également préférée, les copolymères anioniques sont préparés par une réaction de polymérisation qui met également en œuvre :
(a3) au moins un composé de formule (I) :

R¹⁻(OE)ₘ-(OP)ₙ-R² (I)

dans laquelle :
- m et n, identiques ou différents, représentent indépendamment 0 ou un nombre entier ou décimal inférieur à 150, m ou n est différent de 0,
- OE représente indépendamment un groupement CH₂CH₂O,
- OP représente indépendamment un groupement choisi parmi CH(CH₃)CH₂O et CH₂CH(CH₃)O,
- R¹ représente un groupement comprenant au moins une insaturation oléfinique polymérisable, de préférence un groupement acrylate ou un groupement méthacrylate et
- R² représente un groupement C₆-C₄₀-alkyl, linéaire ou ramifié, un groupement phényl, un groupement polyphényl, de préférence un groupement C₁₀-C₃₀-alkyl, linéaire ou ramifié, plus préférentiellement un groupement C₁₂-C₂₂-alkyl, linéaire ou ramifié, ou un groupement comprenant 2 à 5 phényls ou un groupement tristyrylphényl ou un groupement pentastyrylcumylphényl.

De manière également préférée, les copolymères anioniques sont préparés par une réaction de polymérisation qui met également en œuvre :
(a4) au moins un composé choisi parmi l'acide 2-acrylamido-2-méthylpropane sulfonique, l'acide éthoxyméthacrylate sulfonique, méthallyl sulfonate de sodium, styrène sulfonate, hydroxyéthyl-acrylate phosphaté, hydroxypropyl-acrylate phosphaté, hydroxyéthylhexyl-acrylate phosphaté, hydroxyéthyl-méthacrylate phosphaté, hydroxypropyl-méthacrylate phosphaté, hydroxyéthylhexyl-méthacrylate phosphaté, leurs sels et leurs combinaisons.

De manière également préférée, les copolymères anioniques sont préparés par une réaction de polymérisation qui met également en œuvre :
(a5) au moins un composé choisi parmi hydroxyéthyl-acrylate, hydroxypropyl-acrylate, hydroxyéthylhexyl-acrylate, hydroxyéthyl-méthacrylate, hydroxypropyl-méthacrylate, hydroxyéthylhexyl-méthacrylate.

De manière également préférée, les copolymères anioniques sont préparés par une réaction de polymérisation qui met également en œuvre :
(a6) au moins un monomère réticulant ou d'au moins un monomère comprenant au moins deux insaturations oléfiniques.

Les copolymères anioniques particulièrement préférés selon l'invention sont préparés par une réaction de polymérisation qui met en œuvre :
(a1) l'acide acrylique, l'acide méthacrylique ou bien l'acide acrylique et l'acide méthacrylique,
(a2) l'acrylate de méthyle, le méthacrylate de méthyle ou bien l'acrylate de méthyle et le méthacrylate de méthyle,
(a3) d'au moins un composé de formule (I) :

   R¹⁻(OE)ₘ-(OP)ₙ-R² (I)
dans laquelle :
   - m et n, identiques ou différents, représentent indépendamment 0 ou un nombre entier ou décimal inférieur à 150, m ou n est différent de 0,
   - OE représente indépendamment un groupement CH₂CH₂O,
   - OP représente indépendamment un groupement choisi parmi CH(CH₃)CH₂O et CH₂CH(CH₃)O,
   - R¹ représente un groupement un groupement acrylate ou un groupement méthacrylate et
   - R² représente un groupement C₆-C₄₀-alkyl, linéaire ou ramifié, un groupement phényl, un groupement polyphényl, de préférence un groupement C₁₀-C₃₀-alkyl, linéaire ou ramifié, plus préférentiellement un groupement C₁₂-C₂₂-alkyl, linéaire ou ramifié, ou un groupement comprenant 2 à 5 phényls ou un groupement tristyrylphényl ou un groupement pentastyrylcumylphényl.

D'autres copolymères anioniques particulièrement préférés selon l'invention sont préparés par une réaction de polymérisation qui met également en œuvre :
(a1) l'acide acrylique, l'acide méthacrylique ou bien l'acide acrylique et l'acide méthacrylique,
(a2) l'acrylate de méthyle, le méthacrylate de méthyle ou bien l'acrylate de méthyle et le méthacrylate de méthyle,
(a3) d'au moins un composé de formule (I) :

   - R¹⁻(OE)ₘ-(OP)ₙ-R² (I)
dans laquelle :
   - m et n, identiques ou différents, représentent indépendamment 0 ou un nombre entier ou décimal inférieur à 150, m ou n est différent de 0,
   - OE représente indépendamment un groupement CH₂CH₂O,
   - OP représente indépendamment un groupement choisi parmi CH(CH₃)CH₂O et CH₂CH(CH₃)O,
   - R¹ représente un groupement un groupement acrylate ou un groupement méthacrylate et
   - R² représente un groupement C₆-C₄₀-alkyl, linéaire ou ramifié, un groupement phényl, un groupement polyphényl, de préférence un groupement C₁₀-C₃₀-alkyl, linéaire ou ramifié, plus préférentiellement un groupement C₁₂-C₂₂-alkyl, linéaire ou ramifié, ou un groupement comprenant 2 à 5 phényls ou un groupement tristyrylphényl ou un groupement pentastyrylcumylphényl et
(a4) l'acide 2-acrylamido-2-méthylpropane sulfonique.

D'autres copolymères anioniques particulièrement préférés selon l'invention sont préparés par une réaction de polymérisation qui met également en œuvre :
(a1) l'acide acrylique, l'acide méthacrylique ou bien l'acide acrylique et l'acide méthacrylique,
(a2) l'acrylate de méthyle, le méthacrylate de méthyle ou bien l'acrylate de méthyle et le méthacrylate de méthyle,
(a4) l'acide 2-acrylamido-2-méthylpropane sulfonique.

Lors de la préparation du composé modificateur de rhéologie mis en œuvre selon l'invention, il est possible d'utiliser au moins un agent de transfert de chaînes, de préférence choisi parmi les composés mercaptans, en particulier les composés mercaptans comprenant au moins quatre atomes de carbone tels que le butylmercaptan, le n-octylmercaptan, le n-dodécylmercaptan, le *tert*-dodécylmercaptan.

De manière préférée selon l'invention, le mélange (M) comprend de 0,5 à 15 % en poids, de préférence de 1 à 15 % en poids ou de 2 à 12 % en poids, de modificateur de rhéologie. De manière plus préférée selon l'invention, le mélange (M) comprend de 1 à 15 % en poids ou de 2 à 12 % en poids, de modificateur de rhéologie.

De manière avantageuse, la méthode selon l'invention met en œuvre un mélange (M) qui comprend également une base. De préférence, il s'agit d'une base minérale, en particulier une base choisie parmi NaOH, KOH, dérivés ammonium, ammoniaque et leurs combinaisons. Également de préférence, il s'agit d'une base choisi parmi les bases aminées, par exemples triéthanolamine, aminométhylpropanol ou 2-amino-2-méthyl-propanol (AMP) et leurs combinaisons.

De manière également avantageuse, la méthode selon l'invention met en œuvre un mélange (M) qui a un pH supérieur à 5, de préférence supérieur à 5,5, plus préférentiellement supérieur à 6. Également de manière avantageuse, la méthode selon l'invention met en œuvre un mélange (M) qui a un pH inférieur à 12.

De manière plus avantageuse, la méthode selon l'invention met en œuvre un mélange (M) qui a un pH allant de 5 à 12, de préférence de 5,5 à 12, plus préférentiellement de 6 à 12.

Généralement selon l'invention, le mélange (M) ne comprend pas de composé tensio-actif ou bien comprend une quantité de composé tensio-actif, de préférence non-ionique, qui est faible. Alors, la quantité de composé tensio-actif, de préférence non-ionique, peut aller de 0,05 à 10 % en poids ou de 0,05 à 5 % en poids, du poids de mélange (M).

De manière avantageuse selon l'invention, la température de préparation est inférieure au point d'ébullition de la phase hydrophile et inférieure au point d'ébullition de la phase lipophile. De manière également avantageuse selon l'invention, la température de préparation est supérieure au point de fusion de la phase hydrophile et supérieure au point de fusion de la phase lipophile.

De manière préférée, la température de préparation est inférieure au point d'ébullition de la phase hydrophile et inférieure au point d'ébullition de la phase lipophile tout en étant supérieure au point de fusion de la phase hydrophile et supérieure au point de fusion de la phase lipophile.

La méthode selon l'invention comprend l'addition du composé lipophile dans la phase continue en appliquant une contrainte choisie parmi une contrainte de cisaillement et une contrainte élongationnelle. De manière préférée, il s'agit d'une contrainte de cisaillement. Avantageusement, la contrainte de cisaillement ou la contrainte élongationnelle peut être appliquée lors de la préparation du mélange (M), de préférence a une valeur égale ou inférieure à celle appliquée lors de l'addition du composé lipophile.

De manière préférée selon l'invention, la contrainte de cisaillement ou la contrainte élongationnelle va de 300 à 5 000 Pa.

De manière également préférée selon l'invention, la contrainte de cisaillement ou la contrainte élongationnelle va de 100 à 2 000 Pa ou de 300 à 2 000 Pa.

De manière plus préférée selon l'invention, la contrainte de cisaillement ou la contrainte élongationnelle va de 100 à 1 700 Pa ou de 300 à 1 700 Pa.

Selon l'invention, la contrainte est appliquée au moyen d'un dispositif produisant un gradient de cisaillement inférieur à 5 000 s⁻¹.

De manière préférée, le dispositif mis en œuvre peut produire un gradient de cisaillement inférieur à 2 000 s⁻¹ ou inférieur à 1 000 s⁻¹. De manière également préférée, le dispositif mis en œuvre peut produire un gradient de cisaillement allant de 100 à 5 000 s⁻¹ ou allant de 100 à 2 000 s⁻¹ ou bien encore allant de 100 à 1 000 s⁻¹.

De manière plus préférée, le dispositif mis en œuvre peut produire un gradient de cisaillement allant de 200 à 5 000 s⁻¹ ou allant de 200 à 2 000 s⁻¹ ou bien encore allant de 200 à 1 000 s⁻¹, en particulier allant de 200 à 800 s⁻¹,

De manière particulièrement avantageuse, le dispositif utilisé est un mixeur, notamment un mixeur VMI Rayneri, un mixeur Ika ou un mixeur PC Labor System.

La méthode de préparation selon l'invention comprend la préparation d'une phase continue sous la forme du mélange (M) puis l'addition du composé lipophile dans la phase continue en appliquant une contrainte de cisaillement. D'autres étapes complémentaires peuvent également être mises en œuvre lors de la méthode de préparation selon l'invention. Ainsi, de manière avantageuse, la méthode de préparation selon l'invention peut également comprendre la neutralisation de la dispersion (D). De préférence, la neutralisation est réalisée au moyen d'au moins un composé choisi parmi NaOH, KOH, dérivés ammonium, ammoniaque, bases aminées, par exemples triéthanolamine, aminométhylpropanol ou 2-amino-2-méthyl-propanol (AMP) et leurs combinaisons.

Également de manière avantageuse, la méthode de préparation selon l'invention peut également comprendre la coacervation partielle du composé modificateur de rhéologie. De manière préférée, la coacervation partielle du composé modificateur de rhéologie est réalisée par réduction du pH de la dispersion (D), par exemple par réduction du pH à une valeur inférieure à 6,5. La réduction du pH peut être réalisée au moyen d'un composé acide, en particulier au moyen d'au moins un composé acide organique ou minéral, notamment un composé acide choisi parmi acide phosphorique, acide citrique, glucono-lactone, acide lactique, acide salicylique, acide glycolique, acide ascorbique, acide glutamique, acide hydrochlorique, acide acétique, acide D-gluconique, acide sulfonique, acide méthane-sulfonique, acide benzimidazole-sulfonique, acide tartrique, acide 4-aminobenzoique, acide benzoïque, acide sorbique, acide phenylbenzimidazole sulfonique, acide benzylidene camphor sulfonique, acide terephthalylidene dicamphor sulfonique.

Également de manière préférée, la coacervation partielle du composé modificateur de rhéologie est réalisée par augmentation de la force ionique de la dispersion (D). L'augmentation de la force ionique de la dispersion (D) peut être réalisée par addition d'au moins un composé ionisé ou d'au moins un sel, en particulier NaCl, KCl, MgCl₂, CaCl₂, MgSO₄, CaSO₄.

Également de manière préférée, la coacervation partielle du composé modificateur de rhéologie est réalisée par réduction de la solubilité du composé modificateur de rhéologie dans la phase hydrophile. La réduction de solubilité peut être réalisée par addition d'au moins un polymère cationique, en particulier par addition d'au moins un polymère cationique, notamment un polymère cationique choisi parmi polyquaternium 1 à polyquaternium 47 et guars quaternisées, notamment chlorure d'hydroxypropyl-guar triammonium, chlorure de polydiallyldiméthylammonium (polyDADMAC ou polyDDA), chlorure de poly-2-(méthacryloyloxy)éthyl-triméthyl-ammonium (polyMAD quat).

La méthode selon l'invention peut combiner l'une ou l'autre de ces étapes supplémentaires. Par exemple, la méthode selon l'invention peut également comprendre la neutralisation de la dispersion (D) et la coacervation partielle du composé modificateur de rhéologie.

La méthode selon l'invention permet de préparer une dispersion (D) qui est particulièrement avantageuse en tant que telle. Ainsi, l'invention concerne également une dispersion (D) susceptible d'être préparée selon l'invention.

La dispersion (D) selon l'invention peut être utilisée dans de nombreux produits qui sont alors particulièrement avantageux en tant que tels. L'invention concerne donc également un produit comprenant au moins une dispersion (D) selon l'invention.

Du fait qu'ils comprennent une phase lipophile dispersée dans une phase hydrophile continue sous la forme de particules nanométriques, la dispersion (D) selon l'invention et le produit selon l'invention peuvent être mis en œuvre dans de très nombreux domaines techniques.

Ainsi, l'invention concerne l'utilisation d'une dispersion (D) dans un domaine choisi parmi la cosmétique, la peinture, la teinture, l'imprimerie, les encres, la construction, les combustibles, les lubrifiants, les agents anti-mousse, la métallurgie, les fertilisants, la pharmacie, l'agrochimie, les produits phytosanitaires, la détergence, l'alimentation, le cuir, l'enduction, notamment l'enduction textile.

L'invention concerne également l'utilisation d'un produit selon l'invention dans un domaine choisi parmi la cosmétique, la peinture, la teinture, l'imprimerie, les encres, la construction, les combustibles, les lubrifiants, les agents anti-mousse, la métallurgie, les fertilisants, la pharmacie, l'agrochimie, les produits phytosanitaires, la détergence, l'alimentation, le cuir, l'enduction, notamment l'enduction textile.

Les exemples qui suivent permettent d'illustrer les différents aspects de l'invention.

### EXEMPLES

### Exemple 1 : préparation de composés modificateurs de rhéologie selon l'invention

Dans un réacteur de polymérisation, on introduit 474,9 g d'eau bipermutée, 6,51 g de sodium dodecyl sulfate (SDS) et 5,45 g d'alcool tridécylique tri-éthoxylé (Rhodasurf ID 030, Solvay). On place le réacteur sous agitation et on le chauffe à 76°C.

Séparément, dans un bécher on pèse 153 g d'eau bipermutée, 2,28 g de sodium dodecyl sulfate, 0,163 g de n-dodecyl mercaptan, 109,04 d'acrylate d'éthyle (AE) comme composé (a2), 112,5 g d'acide méthacrylique (AMA) comme composé (a1), 45,31 g de méthacrylate de méthyl comme autre composé (a2), 13,445 g de méthacrylate-(OE)25-C16 ramifié comme composé (a3) de formule (I) dans laquelle R¹ représente un groupement méthacrylate, R² représente un groupement C₁₆-alkyl ramifié, m=25 et n=0, 13,445 g de méthacrylate-(OE)30-C₁₂ ramifié comme autre composé (a3) de formule (I) dans laquelle R¹ représente un groupement méthacrylate, R² représente un groupement C₁₂-alkyl ramifié, m=30 et n=0. On place ce mélange sous agitation à l'aide d'un barreau aimanté.

On ajoute simultanément dans le réacteur un mélange contenant 0,925 g de persulfate d'ammonium et 4,07 g d'eau bipermutée et un second mélange composé de 0,093 g de bisulfite de sodium et 4,88 g d'eau bipermutée. Puis, on débute l'injection du mélange qui dure 2 heures. La température est maintenue à 76°C.

On obtient le polymère (P1) selon les proportions présentées dans le tableau 1 dans lequel les valeurs sont des proportions en poids.

De manière analogue, on prépare les polymères (P2), (P3), pour lequel (a3) est un composé de formule (I) dans laquelle R¹ représente un groupement méthacrylate, R² représente un groupement tristyrylphényl (TSP), m=25 et n=0, et (P4) selon les proportions (en g sec pour sec) présentées dans le tableau 1. La quantité totale de monomères est de 100 % en poids et la quantité d'agent de transfert de chaîne est relative à la quantité totale en poids de monomères.

**Tableau 1**

| polymère modificateur de rhéologie | P1 | P2 | P3 | P4 |
|---|---|---|---|---|
| a1 : AMA | 37,92 | 36,74 | 40,31 | 0,49 |
| a1 : AA | / | / | / | 36,99 |
| a2 : MAM | 15,58 | 27,03 | / | / |
| a2 : AE | 37,50 | 27,03 | 49,73 | 53,21 |
| a3 : méthacrylate-(OE)30-C12 ramifié | 4,50 | / | / | / |
| a3 : méthacrylate-(OE)25-C16 ramifié | 4,50 | 9,21 | / | 8,21 |
| a3 : méthacrylate-(OE)25-TSP | / | / | 8,04 | / |
| a4 : AMPS | / | / | 1,92 | 1,09 |
| agent de transfert : n-dodecylmercaptan | 0,06 | 0,29 | / | / |

### Exemple 2 : préparation de dispersions selon l'invention

On prépare un mélange (M) de phase continue hydrophile à partir de polymère modificateur de rhéologie, d'eau et éventuellement d'un composé hydrophile supplémentaire et éventuellement d'un composé tensio-actif non-ionique (TA) choisi parmi Plantaren 2000 N UP (Basf), Sensient LRI (Sensient Cosmetic Technologies), Rhodasurf ID 030 (Solvay), Polysorbate 20 (Sigma-Aldrich) et Disponil G625 (Basf). On ajoute une base et on met sous agitation jusqu'à obtenir un mélange aqueux homogène. Séparément, on prépare le composé lipophile à disperser.

Puis, on introduit, sous agitation réalisée au moyen d'un mixeur, la phase lipophile dans la phase hydrophile continue pour produire une émulsion.

Le cas échéant, on ajoute un agent de coacervation acide ou salin (diluant acide ou diluant salin).

Des dispersions de différents composés lipophiles ou de mélanges de composés lipophiles ont été préparées : résine alkyde 1 (viscosité à 70°C de 500 000 mPa.s), résine alkyde 2 (viscosité à 70°C de 6 550 mPa.s), résine alkyde 3 (viscosité à 70°C de 230 mPa.s), mélange résine alkyde 1 et acide linoléique (viscosité à 70°C de 8 000 mPa.s), huile de tournesol (viscosité à température ambiante de 560 mPa.s), n-octyltriethoxysilane (viscosité à température ambiante de 16,7 mPa.s).

On obtient des dispersions selon l'invention selon les quantités (g) et les caractéristiques présentées dans les tableaux 2 à 7 et dont la taille des particules de composé lipophile dispersées est inférieure à 1 µm.

**Tableau 2**

| Dispersion | | D1 | D2 | D3 | D4 | D5 |
|---|---|---|---|---|---|---|
| matières premières | polymère | P1 | P2 | P2 | P2 | P3 |
| | autre composé ou TA | / | SDS | / | / | / |
| | base | NaOH | NaOH | NaOH | NaOH | NaOH |
| | acide | H₃PO₄ | H₃PO₄ | H₃PO₄ | H₃PO₄ | / |
| | sel | CaCl₂ | CaCl₂ | CaCl₂ | CaCl₂ | / |
| | composé lipophile : résine alkyde | 1 | 1 | 2 | 3 | 1 |
| émulsification | mixeur (à 500 s⁻¹) | VMI Rayneri | | | | |
| | température | 60-70°C | | | | |
| phase continue hydrophile | polymère sec | 4,341 | 4,356 | 4,356 | 4,356 | 4,356 |
| | autre composé sec ou TA sec | / | 0,25 | / | / | / |
| | eau | 47,699 | 45,176 | 45,176 | 45,176 | 45,176 |
| | base sèche | 0,53 | 0,468 | 0,468 | 0,468 | 0,468 |
| | pH | 6 +/- 0,3 | | | | |
| | polymère (% en poids) | 8,26 | 8,67 | 8,71 | 8,71 | 8,71 |
| | contrainte (Pa) | 900 | 300 | 500 | 500 | 300 |
| composé lipophile dispersé | | 77,9 | 75 | 75 | 75 | 75 |
| diluant acide | eau | 29,702 | 25,152 | 25,152 | 25,152 | / |
| | acide | 0,052 | 0,048 | 0,048 | 0,048 | / |
| diluant salin | eau | 14 | 14 | 14 | 14 | / |
| | sel | 0,07 | 0,07 | 0,07 | 0,07 | / |
| taille des particules | D50 % (µm) | 0,9 | 0,8 | 0,6 | 0,5 | 0,9 |
| | D40 % (µm) | 0,5 | 0,4 | 0,5 | 0,5 | 0,5 |
| | D30 % (µm) | 0,4 | 0,3 | 0,5 | 0,5 | 0,4 |

**Tableau 3**

| dispersion | | D6 | D7 | D8 |
|---|---|---|---|---|
| matières premières | polymère | P1 | P2 | P2 |
| | autre composé ou TA | Disponil G625 | | |
| | base | NaOH | AMP | AMP |
| | acide | HCl | H₃PO₄ | acide D-gluconique |
| | sel | / | / | / |
| | composé lipophile : résine alkyde | 1 | 1 | 1 |
| émulsification | mixeur (à 500 s⁻¹) | VMI Rayneri | | PC Laborsystem |
| | température | 60-70°C | | |
| phase continue hydrophile | polymère sec | 5,64 | 3,843 | 19,335 |
| | autre composé sec ou TA sec | 0,4 | 0,88 | 4,4 |
| | eau | 75,06 | 30,4845 | 152,5125 |
| | base sèche | 0,6 | 0,9025 | 4,5125 |
| | pH | 6 +/- 0,3 | | |
| | polymère (% en poids) | 6,90 | 10,64 | 10,70 |
| | contrainte (Pa) | non disponible | | |
| composé lipophile dispersé | | 150 | 75 | 375 |
| diluant acide | eau | 40,048 | 25,0272 | 190 |
| | acide | 0,092 | 0,0428 | 4,15 |
| diluant salin | eau | / | 24 | / |
| | sel | / | 0,07 | / |
| taille des particules | D50 % (µm) | 0,5 | 0,5 | 0,4 |
| | D40 % (µm) | 0,4 | 0,4 | 0,3 |
| | D30 % (µm) | 0,3 | 0,3 | 0,2 |

| | | | | |
|---|---|---|---|---|
| ^{∗}sans résine alkyde, addition progressive | | | | |

**Tableau 4**

| dispersion | | D9 | D10 | D11 |
|---|---|---|---|---|
| matières premières | polymère | P2 | P2 | P2 |
| | autre composé ou TA | Disponil G625 | Polysorbate 20 | acétylcitrate de 2-tributyl |
| | base | AMP | NaOH | AMP |
| | acide | acide ascorbique | / | acide D-gluconique |
| | sel | / | NaCl | / |
| | composé lipophile : résine alkyde | 1 | huile de tournesol^{∗} | 1 |
| émulsification | mixeur (à 500 s⁻¹) | VMI Rayneri | PC Laborsystem | VMI Rayneri |
| | température | 60-70°C | température ambiante | 60-70°C |
| phase continue hydrophile | polymère sec | 3,93 | 22,71 | 3,7992 |
| | autre composé sec ou TA sec | 0,88 | 4,1 | 0,39 |
| | eau | 30 ,3975 | 188,93 | 32,9008 |
| | base sèche | 0,9025 | 2,56 | 0,912 |
| | pH | 6 +/- 0,3 | | |
| | polymère (% en poids) | 10,88 | 10,40 | 10,00 |
| | contrainte (Pa) | non disponible | | |
| composé lipophile dispersé | | 75 | 410 | 75 |
| diluant acide | eau | 38 | / | 38 |
| | acide | 0,435 | / | 0,83 |
| diluant salin | eau | 20 | 450 | / |
| | sel | / | 3,1 | / |
| taille des particules | D50 % (µm) | 0,5 | 0,9 | 0,9 |
| | D40 % (µm) | 0,4 | 0,8 | 0,4 |
| | D30 % (µm) | 0,3 | 0,6 | 0,3 |

| | | | | |
|---|---|---|---|---|
| ^{∗}sans résine alkyde, addition progressive | | | | |

**Tableau 5**

| dispersion | | D12 | D13 | D14 | D15 |
|---|---|---|---|---|---|
| matières premières | polymère | P2 | P2 | P2 | P2 |
| | autre composé ou TA | / | / | Rhodasurf ID 030 | / |
| | base | NaOH | | | |
| | acide | H₃PO₄ | | | / |
| | sel | CaCl₂ | | | NaCl |
| | composé lipophile : résine alkyde | 1 | Acide linoléique | 1 | huile de tournesol ^{∗} |
| émulsification | mixeur (à 500 s⁻¹) | PC Laborsystem | VMI Rayneri | | PC Laborsystem |
| | température | 60-70°C | | température ambiante | |
| phase continue hydrophile | polymère sec | 21,75 | 4,356 | 4,356 | 22,71 |
| | autre composé sec ou TA sec | / | / | 0,7 | / |
| | eau | 225,95 | 45,176 | 45,176 | 188,93 |
| | base sèche | 2,3 | 0,468 | 0,468 | 2,56 |
| | pH | 6 +/- 0,3 | | | |
| | polymère (% en poids) | 8,70 | 8,71 | 8,59 | 10,60 |
| | contrainte (Pa) | 500 | 500 | 1700 | 600 |
| composé lipophile dispersé | | 376,2 | 75 | 75 | 434 |
| diluant acide | eau | 125,26 | 25,152 | 25,152 | / |
| | acide | 0,24 | 0,048 | 0,048 | / |
| diluant salin | eau | 70 | 14 | 14 | 450 |
| | sel | 0,35 | 0,07 | 0,07 | 3 |
| taille des particules | D40 % (µm) | 0,8 | 0,9 | 0,9 | 0,9 |
| | D30 % (µm) | 0,5 | 0,4 | 0,4 | 0,7 |

| | | | | | |
|---|---|---|---|---|---|
| ^{∗} sans résine alkyde, addition progressive | | | | | |

**Tableau 6**

| dispersion | | D16 | D17 | D18 |
|---|---|---|---|---|
| matières premières | polymère | P2 | P2 | P2 |
| | autre composé ou TA | / | / | / |
| | base | NaOH | | |
| | acide | H₃PO₄ | | / |
| | sel | / | CaCl₂ | / |
| | composé lipophile : résine alkyde | 1 | 1 | n-octyltriethoxysilane^{∗} |
| émulsification | mixeur (à 500 s⁻¹) | PC Laborsystem | | VMI Rayneri |
| | température | 60-70°C | | température ambiante |
| phase continue hydrophile | polymère sec | 21,75 | 24,06 | 2,28 |
| | autre composé sec ou TA sec | / | / | / |
| | eau | 225,95 | 250,59 | 24,84 |
| | base sèche | 2,3 | 2,55 | 0,26 |
| | pH | 6 +/- 0,3 | | |
| | polymère (% en poids) | 8,70 | 8,68 | 8,33 |
| | contrainte (Pa) | 500 | 500 | 300 |
| composé lipophile dispersé | | 377,7 | 521 | 31,8 |
| diluant acide | eau | 124,76 | 244,132 | / |
| | acide | 0,24 | 0,268 | / |
| diluant salin | eau | / | 70 | / |
| | sel | / | 0,42 | / |
| diluant | eau | / | / | 31 |
| taille des particules | D30 % (µm) | 0,8 | 0,9 | 0,8 |

| | | | | |
|---|---|---|---|---|
| ^{∗} sans résine alkyde, addition progressive | | | | |

**Tableau 7**

| dispersion | | D19 | D20 | D21 |
|---|---|---|---|---|
| matières premières | polymère | P4 | P2 | P2 |
| | autre composé ou TA | / | Plantaren 2000 N UP | Sensient LRI |
| | base | NaOH | | |
| | acide | H₃PO₄ | / | / |
| | sel | CaCl₂ | NaCl | |
| | composé lipophile : résine alkyde | 1 | huile de tournesol^{∗} | |
| émulsification | mixeur (à 500 s⁻¹) | PC Laborsystem | | |
| | température | 60-70°C | température ambiante | |
| phase continue hydrophile | polymère sec | 22,477 | 23,34 | 22,71 |
| | autre composé sec ou TA sec | / | 2 | 1,19 |
| | eau | 238,943 | 187,16 | 188,93 |
| | base sèche | 3,78 | 2,6 | 6,4 |
| | pH | 6 +/- 0,3 | | |
| | polymère (% en poids) | 8,48 | 10,85 | 10,54 |
| | contrainte (Pa) | non disponible | | |
| composé lipophile dispersé | | 376,2 | 431,1 | 447 |
| diluant acide | eau | 206,712 | / | / |
| | acide | 4,388 | / | / |
| diluant salin | eau | 70 | 453,1 | 450 |
| | sel | 0,35 | 3 | 3,2 |
| taille des particules | D30 % (µm) | 0,9 | 0,8 | 0,8 |

| | | | | |
|---|---|---|---|---|
| ^{∗} sans résine alkyde, addition progressive | | | | |

La méthode selon l'invention permet de préparer aisément, au moyen d'un mixeur usuel produisant un faible gradient de cisaillement, des émulsions de différents composés lipophiles dispersés dans une phase continue hydrophile pour lesquelles la taille des particules de composé lipophile est bien inférieure à 1 µm.

## Revendications

1. Méthode de préparation d'une dispersion (D) comprenant :
• une phase continue hydrophile comprenant au moins un composé hydrophile et au moins un composé modificateur de rhéologie du composé hydrophile, et choisi parmi les copolymères anioniques, et
• une phase lipophile dispersée dans la phase continue sous la forme de particules nanométriques,
comprenant :
- la préparation d'un mélange (M) comprenant le composé hydrophile et le composé modificateur de rhéologie du composé hydrophile,
- l'addition du composé lipophile dans la phase continue en appliquant, au moyen d'un dispositif produisant un gradient de cisaillement inférieur à 5 000 s⁻¹, une contrainte choisie parmi une contrainte de cisaillement allant de 100 à 5 000 Pa et une contrainte élongationnelle allant de 100 à 5 000 Pa.

2. Méthode selon la revendication 1 pour laquelle :
• la phase continue hydrophile a une viscosité allant de 20 à 50 000 mPa.s, de préférence de 100 à 50 000 mPa.s ou de 100 à 20 000 mPa.s ou
• la dispersion (D) :
∘ a une viscosité allant de 20 à 50 000 mPa.s, de préférence de 100 à 50 000 mPa.s ou de 100 à 20 000 mPa.s ou
∘ est une émulsion ou
∘ comprend de 0,1 à 75 % en poids ou de 0,3 à 75 % en poids ou de 1 à 75 % en poids, de préférence de 0,1 à 70 % en poids ou de 0,3 à 70 % en poids ou de 1 à 70 % en poids, plus préférentiellement de 0,1 à 65 % en poids ou de 0,3 à 65 % en poids ou de 1 à 65 % en poids, également plus préférentiellement de 0,1 à 60 % en poids ou de 0,3 à 60 % en poids ou de 1 à 60 % en poids, de phase lipophile dispersée par rapport à la quantité totale en poids de phase hydrophile continue et de phase lipophile dispersée.

3. Méthode selon l'une des revendications 1 ou 2 pour laquelle le composé hydrophile est choisi parmi l'eau seule ou en mélange avec au moins un composé choisi parmi glycérol, polyglycérols, glycols, par exemple propylèneglycol, butylèneglycol, composés humectants, par exemple des composés humectants pour composition cosmétique, dérivés de sucres, par exemple xylytol, maltilol, agents de coalescence, par exemple des polyalkylène-glycols de faible masse moléculaire, notamment polyéthylèneglycol, butyldiglycol.

4. Méthode selon l'une des revendications 1 à 3 pour laquelle le composé modificateur de rhéologie :
• a un pH supérieur à 5, de préférence supérieur à 5,5, plus préférentiellement supérieur à 6 ou
• est choisi parmi les copolymères ASE, les copolymères HASE et leurs combinaisons, de préférence parmi les copolymères anioniques préparés par réaction de polymérisation :
(a1) d'au moins un monomère anionique comprenant au moins une insaturation oléfinique polymérisable, de préférence un monomère anionique comprenant au moins une insaturation oléfinique polymérisable et au moins une fonction acide carboxylique, de préférence le monomère anionique est choisi parmi l'acide acrylique, l'acide méthacrylique, l'acide maléique, l'anhydride maléique, l'acide itaconique, l'acide crotonique, un sel d'acide acrylique, un sel d'acide méthacrylique, un sel d'acide maléique, un sel d'anhydride maléique, un sel d'acide itaconique, un sel d'acide crotonique et leurs combinaisons, bien plus préférentiellement l'acide acrylique ou l'acide méthacrylique et
(a2) d'au moins un ester d'un composé dérivé d'un acide choisi parmi l'acide acrylique, l'acide méthacrylique, l'acide maléique, l'anhydride maléique, l'acide itaconique et l'acide crotonique, de préférence un ester de l'acide acrylique ou un ester de l'acide méthacrylique, de préférence choisi parmi acrylate de méthyle, acrylate d'éthyle, acrylate de propyle, acrylate de butyle, acrylate d'éthylhexyle, méthacrylate de méthyle, méthacrylate d'éthyle, méthacrylate de propyle, méthacrylate de butyle, méthacrylate d'éthylhexyle et leurs combinaisons, éventuellement
(a3) d'au moins un composé de formule (I) :
R¹⁻(OE)ₘ-(OP)ₙ-R² (I)
dans laquelle :
- m et n, identiques ou différents, représentent indépendamment 0 ou un nombre entier ou décimal inférieur à 150, m ou n est différent de 0,
- OE représente indépendamment un groupement CH₂CH₂O,
- OP représente indépendamment un groupement choisi parmi CH(CH₃)CH₂O et CH₂CH(CH₃)O,
- R¹ représente un groupement comprenant au moins une insaturation oléfinique polymérisable, de préférence un groupement acrylate ou un groupement méthacrylate et
- R² représente un groupement C₆-C₄₀-alkyl, linéaire ou ramifié, un groupement phényl, un groupement polyphényl, de préférence un groupement C₁₀-C₃₀-alkyl, linéaire ou ramifié, plus préférentiellement un groupement C₁₂-C₂₂-alkyl, linéaire ou ramifié, ou un groupement comprenant 2 à 5 phényls ou un groupement tristyrylphényl ou un groupement pentastyrylcumylphényl, éventuellement
(a4) d'au moins un composé choisi parmi l'acide 2-acrylamido-2-méthylpropane sulfonique, l'acide éthoxyméthacrylate sulfonique, méthallyl sulfonate de sodium, styrène sulfonate hydroxyéthyl-acrylate phosphaté, hydroxypropyl-acrylate phosphaté, hydroxyéthylhexyl-acrylate phosphaté, hydroxyéthyl-méthacrylate phosphaté, hydroxypropyl-méthacrylate phosphaté, hydroxyéthylhexyl-méthacrylate phosphaté, leurs sels et leurs combinaisons, éventuellement (a5) d'au moins un composé choisi parmi hydroxyéthyl-acrylate, hydroxypropyl-acrylate, hydroxyéthylhexyl-acrylate, hydroxyéthyl-méthacrylate, hydroxypropyl-méthacrylate, hydroxyéthylhexyl-méthacrylate et également éventuellement (a6) d'au moins un monomère réticulant ou d'au moins un monomère comprenant au moins deux insaturations oléfiniques.

5. Méthode selon l'une des revendications 1 à 4 pour laquelle le mélange (M) :
• comprend également une base, de préférence une base minérale, en particulier une base choisie parmi NaOH, KOH, dérivés ammonium, ammoniaque, bases aminées, par exemples triéthanolamine, aminométhylpropanol ou 2-amino-2-méthyl-propanol (AMP) et leurs combinaisons ou
• a un pH supérieur à 5, de préférence supérieur à 5,5, plus préférentiellement supérieur à 6 ou
• a un pH inférieur à 12 ou
• a un pH allant de 5 à 12, de préférence de 5,5 à 12, plus préférentiellement de 6 à 12 ou
• ne comprend pas de composé tensio-actif ou bien comprend une faible quantité, par exemple une quantité de composé tensio-actif allant de 0,05 à 10 % en poids ou allant de 0,05 à 5 % en poids, du poids du mélange (M) ou bien une quantité de composé tensio-actif non-ionique allant de 0,05 à 10 % en poids ou allant de 0,05 à 5 % en poids, du poids du mélange (M).

6. Méthode selon l'une des revendications 1 à 5 pour laquelle le mélange (M) comprend de 0,5 à 15 % en poids, de préférence de 1 à 15 % en poids ou de 2 à 12 % en poids, de modificateur de rhéologie.

7. Méthode selon l'une des revendications 1 à 6 pour laquelle :
• la température de préparation est inférieure au point d'ébullition de la phase hydrophile et inférieure au point d'ébullition de la phase lipophile ou
• la température de préparation est supérieure au point de fusion de la phase hydrophile et supérieure au point de fusion de la phase lipophile ou
• la température de préparation est inférieure au point d'ébullition de la phase hydrophile et inférieure au point d'ébullition de la phase lipophile tout en étant supérieure au point de fusion de la phase hydrophile et supérieure au point de fusion de la phase lipophile.

8. Méthode selon l'une des revendications 1 à 7 pour laquelle la dispersion est une émulsion de la phase dispersée lipophile dans la phase continue hydrophile.

9. Méthode selon l'une des revendications 1 à 8 pour laquelle la contrainte de cisaillement ou la contrainte élongationnelle :
• va de 300 à 5 000 Pa, de préférence de 100 à 2 000 Pa ou de 300 à 2 000 Pa, plus préférentiellement de 100 à 1 700 Pa ou de 300 à 1 700 Pa ou
• est également appliquée lors de la préparation du mélange (M), de préférence a une valeur égale ou inférieure à celle appliquée lors de l'addition du composé lipophile ou
• la contrainte est appliquée au moyen d'un dispositif produisant un gradient de cisaillement inférieur à 2 000 s⁻¹ ou inférieur à 1 000 s⁻¹ ou au moyen d'un dispositif produisant un gradient de cisaillement allant de 100 à 5 000 s⁻¹ ou allant de 100 à 2 000 s⁻¹ ou bien encore allant de 100 à 1 000 s⁻¹, de préférence allant de 200 à 5 000 s⁻¹ ou de 200 à 2 000 s⁻¹ ou bien de 200 à 1 000 s⁻¹, en particulier allant de 200 à 800 s⁻¹,

10. Méthode selon l'une des revendications 1 à 9 pour laquelle les particules de phase lipophile dispersées ont une taille moyenne (mesurée par diffraction de la lumière) submicronique, de préférence au moins 30 % en volume ou au moins 40 % en volume, de préférence au moins 50 % en volume, des particules de phase lipophile dispersée ont une taille moyenne nanométrique ou submicronique, de manière plus préférée au moins 30 % en volume ou au moins 40 % en volume, plus préférentiellement au moins 50 % en volume, des particules de phase lipophile dispersée ont une taille moyenne allant de 50 à 999 nm ou de 100 à 999 nm ou encore de 50 à 990 nm ou de 500 à 990 nm.

11. Méthode selon l'une des revendications 1 à 10 comprenant également :
• la neutralisation de la dispersion (D), de préférence au moyen d'au moins un composé choisi parmi NaOH, KOH, dérivés ammonium, ammoniaque, bases aminées, par exemples triéthanolamine, aminométhylpropanol ou 2-amino-2-méthyl-propanol (AMP) et leurs combinaisons ou
• la coacervation partielle du composé modificateur de rhéologie, de préférence :
∘ par réduction du pH de la dispersion (D), par exemple par réduction du pH à une valeur inférieure à 6,5, notamment au moyen d'un composé acide, en particulier au moyen d'au moins un composé acide organique ou minéral, notamment un composé acide choisi parmi acide phosphorique, acide citrique, glucono-lactone, acide lactique, acide salicylique, acide glycolique, acide ascorbique, acide glutamique, acide hydrochlorique, acide acétique, acide D-gluconique, acide sulfonique, acide méthane-sulfonique, acide benzimidazole-sulfonique, acide tartrique, acide 4-aminobenzoique, acide benzoïque, acide sorbique, acide phenylbenzimidazole sulfonique, acide benzylidene camphor sulfonique, acide terephthalylidene dicamphor sulfonique ou
∘ par augmentation de la force ionique de la dispersion (D), par exemple par addition d'au moins un composé ionisé ou d'au moins un sel, en particulier NaCl, KCl, MgCl₂, CaCl₂, MgSO₄, CaSO₄ ou
∘ par réduction de la solubilité du composé modificateur de rhéologie dans la phase hydrophile, par exemple par addition d'au moins un polymère cationique, en particulier par addition d'au moins un polymère cationique, notamment un polymère cationique choisi parmi polyquaternium 1 à polyquaternium 47 et guars quaternisées, notamment chlorure d'hydroxypropyl-guar triammonium, chlorure de polydiallyldiméthylammonium (polyDADMAC ou polyDDA), chlorure de poly-2-(méthacryloyloxy)éthyl-triméthyl-ammonium (polyMAD quat).

12. Dispersion (D) susceptible d'être préparée selon l'une des revendications 1 à 11.

13. Produit comprenant au moins une dispersion selon la revendication 12.

14. Utilisation d'une dispersion (D) selon la revendication 12 ou d'un produit selon la revendication 13 dans un domaine choisi parmi la cosmétique, la peinture, la teinture, l'imprimerie, les encres, la construction, les combustibles, les lubrifiants, les agents anti-mousse, la métallurgie, les fertilisants, la pharmacie, l'agrochimie, les produits phytosanitaires, la détergence, l'alimentation, le cuir, l'enduction, notamment l'enduction textile.

## Patentansprüche

1. Verfahren zur Herstellung einer Dispersion (D), umfassend:
• eine kontinuierliche hydrophile Phase mit mindestens einer hydrophilen Verbindung und mindestens einer rheologiemodifizierenden Verbindung der hydrophilen Verbindung, ausgewählt aus den anionischen Copolymeren, und
• eine lipophile Phase, die in der kontinuierlichen Phase in Form von Nanopartikeln dispergiert wird,
umfassend:
- Herstellen einer Mischung (M) aus der hydrophilen Verbindung und der rheologiemodifizierenden Verbindung der hydrophilen Verbindung,
- Hinzufügen der lipophilen Verbindung zur kontinuierlichen Phase durch Anwenden, über eine Vorrichtung mit Bereitstellung einer Scherrate unter 5.000 s⁻¹, einer Beanspruchung, ausgewählt aus einer Scherbeanspruchung zwischen 100 und 5.000 Pa und einer Dehnbeanspruchung zwischen 100 und 5.000 Pa.

2. Verfahren nach Anspruch 1, wobei:
• die kontinuierliche hydrophile Phase eine Viskosität zwischen 20 und 50.000 mPa.s, vorzugsweise zwischen 100 und 50.000 mPa.s oder zwischen 100 und 20.000 mPa.s, hat, oder
• die Dispersion (D):
∘ eine Viskosität zwischen 20 und 50.000 mPa.s, vorzugsweise zwischen 100 und 50.000 mPa.s oder zwischen 100 und 20.000 mPa.s, hat, oder
∘ eine Emulsion ist, oder
∘ 0,1 bis 75 Gew.-% oder 0,3 bis 75 Gew.-% oder 1 bis 75 Gew.-%, vorzugsweise 0,1 bis 70 Gew.-% oder 0,3 bis 70 Gew.-% oder 1 bis 70 Gew.-%, noch mehr bevorzugt 0,1 bis 65 Gew.-% oder 0,3 bis 65 Gew.-% oder 1 bis 65 Gew.-%, auch noch mehr bevorzugt 0,1 bis 60 Gew.-% oder 0,3 bis 60 Gew.-% oder 1 bis 60 Gew.-%, an lipophiler Phase umfasst, die bezogen auf die gesamte Gewichtsmenge der kontinuierlichen hydrophilen Phase und der dispergierten lipophilen Phase dispergiert wird.

3. Verfahren nach einem der Ansprüche 1 oder 2, wobei die hydrophile Verbindung ausgewählt ist aus Wasser allein oder Wasser in Mischung mit mindestens einer Verbindung, ausgewählt aus Glycerin, Polyglycerin, Glycol, zum Beispiel Propylengylcol, Butylenglycol, Feuchthalteverbindungen, beispielsweise Feuchthalteverbindungen für eine kosmetische Zusammensetzung, Zuckerderivaten, beispielsweise Xylit, Maltilol, Koaleszenzmittel, zum Beispiel Polyalkylenglycol mit niedriger molekularer Masse, insbesondere Polyethylenglycol, Butyldiglycol.

4. Verfahren nach einem der Ansprüche 1 bis 3, wobei die rheologiemodifizierende Verbindung:
• einen pH-Wert von über 5, vorzugsweise von über 5,5, noch mehr bevorzugt von über 6, hat, oder
• ausgewählt ist aus den Copolymeren ASE, Copolymeren HASE und deren Kombinationen, vorzugsweise aus den anionischen Copolymeren, die hergestellt werden durch eine Polymerisationsreaktion von:
(a1) mindestens einem anionischen Monomer mit mindestens einer polymerisierbaren olefinischen Ungesättigtheit, vorzugsweise einem anionischen Monomer mit mindestens einer polymerisierbaren olefinischen Ungesättigtheit und mindestens einer Carbonsäurefunktion, noch mehr bevorzugt einem anionischen Monomer ausgewählt aus Acrylsäure, Methacrylsäure, Maleinsäure, Maleinanhydrid, Itaconsäure, Crotonsäure, einem Acrylsäuresalz, einem Methacrylsäuresalz, einem Maleinsäuresalz, einem Maleinanhydridsalz, einem Itaconsäuresalz, einem Crotonsäuresalz und Kombinationen davon, noch viel mehr bevorzugt Acrylsäure oder Methacrylsäure, und
(a2) mindestens einem Ester einer Säurederivatverbindung, ausgewählt aus Acrylsäure, Methacrylsäure, Maleinsäure, Maleinanhydrid, Itaconsäure und Crotonsäure, vorzugsweise einem Ester der Acrylsäure oder einem Ester der Methacrylsäure, vorzugsweise ausgewählt aus Methylacrylat, Ethylacrylat, Propylacrylat, Butylacrylat, Ethylhexylacrylat, Methylmethacrylat, Ethylmethacrylat, Propylmethacrylat, Butylmethacrylat, Ethylhexylmethacrylat und Kombinationen davon, eventuell
(a3) mindestens einer Verbindung mit der Formel (I):
R¹⁻(OE)ₘ-(OP)ₙ-R² (I)
wobei:
- m und n, die gleich oder unterschiedlich sein können, unabhängig 0 oder eine Ganzzahl oder eine Dezimalzahl unter 150 darstellen, wobei m oder n ungleich 0 ist,
- OE unabhängig eine CH₂CH₂O-Gruppe darstellt,
- OP unabhängig eine Gruppe darstellt, die ausgewählt ist aus CH(CH₃)CH₂O und CH₂CH(CH₃)O,
- R¹ eine Gruppe mit mindestens einer polymerisierbaren olefinischen Ungesättigtheit darstellt, vorzugsweise eine Acrylat-Gruppe oder Methacrylat-Gruppe, und
- R² eine lineare oder verzweigte C₆-C₄₀-Alkylgruppe, eine Phenylgruppe, eine Polyphenylgruppe darstellt, vorzugsweise eine lineare oder verzweigte C₁₀-C₃₀-Alkylgruppe, noch mehr bevorzugt eine lineare oder verzweigte C₁₂-C₂₂-Alkylgruppe, oder eine Gruppe mit 2 bis 5 Phenylen oder eine Tristyrylphenylgruppe oder eine Pentastyrylcumylphenylgruppe, eventuell
(a4) mindestens einer Verbindung, ausgewählt aus 2-Acrylamido-2-methylpropansulfonsäure, Ethoxymethacrylatsulfonsäure, Natriummethallylsulfonat, phosphatiertem Hydroxyethylacrylat-Styrolsulfonat, phosphatiertem Hydroxypropylacrylat, phosphatiertem Hydroxyethylhexylacrylat, phosphatiertem Hydroxyethylmethacrylat, phosphatiertem Hydroxypropylmethacrylat, phosphatiertem Hydroxyethylhexylmethacrylat, deren Salzen und Kombinationen davon, eventuell
(a5) mindestens einer Verbindung, ausgewählt aus Hydroxyethylacrylat, Hydroxypropylacrylat, Hydroxyethylhexylacrylat, Hydroxyethylmethacrylat, Hydroxypropylmethacrylat, Hydroxyethylhexylmethacrylat und ebenfalls eventuell
(a6) mindestens einem vernetzenden Monomer oder mindestens einem Monomer, das mindestens zwei olefinische Ungesättigtheiten hat.

5. Verfahren nach einem beliebigen der Ansprüche 1 bis 4, wobei die Mischung (M):
• ebenfalls eine Base umfasst, vorzugsweise eine anorganische Base, insbesondere eine Base ausgewählt aus NaOH, KOH, Ammoniumderivaten, Ammoniak, Aminbasen, zum Beispiel Triethanolamin, Aminomethylpropanol oder 2-Amino-2-Methylpropanol (AMP) und deren Kombinationen, oder
• einen pH-Wert von über 5, vorzugsweise von über 5,5, noch mehr bevorzugt von über 6, hat, oder
• einen pH-Wert von unter 12 hat, oder
• einen pH-Wert von zwischen 5 und 12, vorzugsweise zwischen 5,5 und 12, noch mehr bevorzugt zwischen 6 und 12 hat, oder
• keine tensioaktive Verbindung oder eine tensioaktive Verbindung in geringer Menge hat, beispielsweise eine Menge an tensioaktiver Verbindung zwischen 0,05 und 10 Gew.-% oder zwischen 0,05 und 5 Gew.-% bezogen auf das Gewicht der Mischung (M) oder eine Menge an nichtionischer tensioaktiver Verbindung zwischen 0,05 und 10 Gew.-% oder zwischen 0,05 und 5 Gew.-% bezogen auf das Gewicht der Mischung (M).

6. Verfahren nach einem der Ansprüche 1 bis 5, wobei die Mischung (M) 0,5 bis 15 Gew.-%, vorzugsweise 1 bis 15 Gew.-% oder 2 bis 12 Gew.-%, an Rheologie-Modifikationsmittel enthält.

7. Verfahren nach einem der Ansprüche 1 bis 6, wobei:
• die Herstellungstemperatur unter dem Siedepunkt der hydrophilen Phase und unter dem Siedepunkt der lipophilen Phase liegt, oder
• die Herstellungstemperatur über dem Schmelzpunkt der hydrophilen Phase und über dem Schmelzpunkt der lipophilen Phase liegt, oder
• die Herstellungstemperatur unter dem Siedepunkt der hydrophilen Phase und unter dem Siedepunkt der lipophilen Phase und gleichzeitig über dem Schmelzpunkt der hydrophilen Phase und über dem Schmelzpunkt der lipophilen Phase liegt.

8. Verfahren nach einem der Ansprüche 1 bis 7, wobei die Dispersion eine Emulsion der dispergierten lipophilen Phase in der kontinuierlichen hydrophilen Phase ist.

9. Verfahren nach einem der Ansprüche 1 bis 8, wobei die Scherbeanspruchung oder die Dehnbeanspruchung:
• zwischen 300 und 5.000 Pa liegt, vorzugsweise zwischen 100 und 2.000 Pa oder zwischen 300 und 2.000 Pa, noch mehr bevorzugt zwischen 100 und 1.700 Pa oder zwischen 300 und 1.700 Pa, oder
• ebenfalls bei Herstellung der Mischung (M) vorzugsweise bei einem Wert angewendet wird, der kleiner oder gleich als der Wert ist, der beim Hinzufügen der lipophilen Verbindung angewendet wird, oder
• wobei die Beanspruchung angewendet wird über eine Vorrichtung mit Bereitstellung einer Scherrate von weniger als 2.000 s⁻¹ oder weniger als 1.000 s⁻¹ oder über eine Vorrichtung mit Bereitstellung einer Scherrate von 100 bis 5.000 s⁻¹ oder von 100 bis 2.000 s⁻¹ oder aber von 100 bis 1.000 s⁻¹, vorzugsweise von 200 bis 5.000 s⁻¹ oder von 200 bis 2.000 s⁻¹ oder von 200 bis 1.000 s⁻¹, insbesondere von 200 bis 800 s⁻¹.

10. Verfahren nach einem der Ansprüche 1 bis 9, wobei die dispergierten Partikel der lipophilen Phase eine Durchschnittsgröße (gemessen mittels Lichtbeugung) im Submikrometerbereich, vorzugsweise von mindestens 30 Vol.-% oder mindestens 40 Vol.-%, noch mehr bevorzugt von mindestens 50 Vol.-%, dispergierte Partikel der lipophilen Phase eine Durchschnittsgröße im Nanometer- oder Submikrometerbereich, vorzugsweise von mindestens 30 Vol-% oder mindestens 40 Vol-%, noch mehr bevorzugt von mindestens 50 Vol-%, dispergierte Partikel der lipophilen Phase eine Durchschnittsgröße von 50 bis 999 nm bzw. 100 bis 999 nm oder 50 bis 990 nm bzw. 500 bis 990 nm haben.

11. Verfahren nach einem der Ansprüche 1 bis 10, des Weiteren umfassend:
• das Neutralisieren der Dispersion (D), vorzugsweise mithilfe mindestens einer Verbindung ausgewählt aus NaOH, KOH, Ammoniumderivaten, Ammoniak, Aminbasen, zum Beispiel Triethanolamin, Aminomethylpropanol oder 2-Amino-2-methylpropanol (AMP) und deren Kombinationen, oder
• die teilweise Koazervation der rheologiemodifizierenden Verbindung, vorzugsweise:
∘ durch Reduzieren des pH-Werts der Dispersion (D), beispielsweise durch Reduzierung des pH-Werts auf weniger als 6,5, insbesondere mithilfe einer Säureverbindung, vor allem mithilfe mindestens einer organischen oder anorganischen Säureverbindung, insbesondere einer Säureverbindung ausgewählt aus Phosphorsäure, Zitronensäure, Gluconolacton, Milchsäure, Salicylsäure, Glycolsäure, Ascorbinsäure, Glutaminsäure, Salzsäure, Essigsäure, D-Gluconsäure, Sulfonsäure, Methansulfonsäure, Benzimidazolsulfonsäure, Weinsäure, 4-Aminobenzoesäure, Benzoesäure, Sorbinsäure, Phenylbenzimidazolsulfonsäure, Benzylidencamphersulfonsäure, Terephthalylidendicamphersulfonsäure, oder
∘ durch Erhöhen der Ionenkonzentration der Dispersion (D), zum Beispiel durch Hinzufügen mindestens einer ionisierten Verbindung oder mindestens eines Salzes, insbesondere NaCl, KCl, MgCl₂, CaCl₂,MgSO₄, CaSO₄, oder
∘ durch Reduzieren der Löslichkeit der rheologiemodifizierenden Verbindung in der hydrophilen Phase, zum Beispiel durch Hinzufügen mindestens eines kationischen Polymers, insbesondere durch Hinzufügen mindestens eines kationischen Polymers, vor allem ein kationisches Polymer ausgewählt aus Polyquaternium 1 bis Polyquaternium 47 und quaternisierten Guarverbindungen, vor allem Hydroxypropylguartriammoniumchlorid, Polydiallyldimethylammoniumchlorid (polyDADMAC sowie polyDDA), Poly-2-(methacryloyloxy)ethyltrimethylammoniumchlorid (polyMAD quat).

12. Dispersion (D), die nach einem der Ansprüche 1 bis 11 hergestellt werden kann.

13. Produkt, bestehend aus mindestens einer Dispersion nach Anspruch 12.

14. Verwendung einer Dispersion (D) nach Anspruch 12 oder eines Produkts nach Anspruch 13 in einer Branche, ausgewählt aus Kosmetik, Malerei, Farbstoffen, Druckerei, Tinten, Bau, Brennstoffen, Schmierstoffen, Antischaummitteln, Metallverarbeitung, Düngemitteln, Pharmazie, Agrochemie, Pflanzenschutzmitteln, Waschmitteln, Lebensmitteln, Leder, Beschichtungen, insbesondere Textilbeschichtungen.

## Claims

1. A method of preparing a dispersion (D) comprising:
• a continuous hydrophilic phase comprising at least one hydrophilic compound and at least one rheology-modifying compound of the hydrophilic compound, and chosen among the anionic copolymers, and
• a lipophilic phase dispersed in the continuous phase in the form of nanometric particles,
comprising:
- the preparation of a mixture (M) comprising the hydrophilic compound and the rheology-modifying compound of the hydrophilic compound,
- the addition of the lipophilic compound in the continuous phase by applying, using a device producing a shear gradient of less than 5,000 s⁻¹, a stress chosen among a shear stress ranging from 100 to 5,000 Pa and an extensional stress ranging from 100 to 5,000 Pa.

2. The method according to claim 1, wherein:
• the continuous hydrophilic phase has a viscosity ranging from 20 to 50,000 mPa.s, preferably from 100 to 50,000 mPa.s or from 100 to 20,000 mPa.s or
• the dispersion (D):
∘ has a viscosity ranging from 20 to 50,000 mPa.s, preferably from 100 to 50,000 mPa.s or from 100 to 20,000 mPa.s or
∘ is an emulsion or
∘ comprises from 0.1 to 75% by weight or from 0.3 to 75% by weight or from 1 to 75% by weight, preferably from 0.1 to 70% by weight or from 0.3 to 70% by weight or from 1 to 70% by weight, more preferentially from 0.1 to 65% by weight or from 0.3 to 65% by weight or from 1 to 65% by weight, also more preferentially from 0.1 to 60% by weight or from 0.3 to 60% by weight or from 1 to 60% by weight, of dispersed lipophilic phase relative to the total amount by weight of continuous hydrophilic phase and of dispersed lipophilic phase.

3. The method according to one of claims 1 or 2, wherein the hydrophilic compound is chosen among water alone or in combination with at least one compound chosen among glycerol, polyglycerols, glycols, for example propylene glycol, butylene glycol, moistening compounds, for example moistening compounds for cosmetic compositions, sugar derivatives, for example xylitol, maltilol, coalescent agents, for example polyalkylene glycols with low molecular mass, in particular polyethylene glycol, butyl diglycol.

4. The method according to any of claims 1 to 3, wherein the rheology-modifying compound:
• has a pH greater than 5, preferably greater than 5.5, more preferentially greater than 6 or
• is chosen among the ASE copolymers, HASE copolymers and combinations thereof, preferably among the anionic copolymers prepared by polymerisation reaction:
(a1) of at least one anionic monomer comprising at least one polymerisable olefinic unsaturation, preferably an anionic monomer comprising at least one polymerisable olefinic unsaturation and at least one carboxylic acid group, preferably the anionic monomer is chosen among acrylic acid, methacrylic acid, maleic acid, maleic anhydride, itaconic acid, crotonic acid, an acrylic acid salt, a methacrylic acid salt, a maleic acid salt, a maleic anhydride salt, an itaconic acid salt, a crotonic acid salt and combinations thereof, much more preferentially acrylic acid or methacrylic acid and
(a2) of at least one ester of a compound derived from an acid chosen among acrylic acid, methacrylic acid, maleic acid, maleic anhydride, itaconic acid and crotonic acid, preferably an acrylic acid ester or a methacrylic acid ester, preferably chosen among methyl acrylate, ethyl acrylate, propyl acrylate, butyl acrylate, ethyl hexyl acrylate, methyl methacrylate, ethyl methacrylate, propyl methacrylate, butyl methacrylate, ethyl hexyl methacrylate, and combinations thereof, optionally (a3) of at least one compound of formula (I):
R¹-(EO)ₘ-(PO)ₙ-R² (I)
wherein:
- m and n, identical or different, independently represent 0 or an integer or decimal less than 150, m or n is different from 0,
- EO independently represents a CH₂CH₂O group,
- PO independently represents a group chosen among CH(CH₃)CH₂O and CH₂CH(CH₃)O,
- R¹ represents a group comprising at least one polymerisable olefinic unsaturation, preferably an acrylate group or a methacrylate group and
- R² represents a straight or branched C₆-C₄₀-alkyl group, a phenyl group, a polyphenyl group, preferably a straight or branched C₁₀-C₃₀-alkyl group, more preferentially a straight or branched C₁₂-C₂₂-alkyl group, or a group comprising 2 to 5 phenyls or a tristyrylphenyl group or a pentastyrylcumylphenyl group, optionally
(a4) of at least one compound chosen among 2-acrylamido-2-methylpropane sulphonic acid, ethoxymethacrylate sulphonic acid, sodium methallyl sulphonate, styrene sulphonate, hydroxyethyl acrylate phosphate, hydroxypropyl acrylate phosphate, hydroxyethylhexyl acrylate phosphate, hydroxyethyl methacrylate phosphate, hydroxypropyl methacrylate phosphate, hydroxyethylhexyl methacrylate phosphate, their salts and combinations thereof, optionally
(a5) of at least one compound chosen among hydroxyethyl-acrylate, hydroxypropyl-acrylate, hydroxyethylhexyl-acrylate, hydroxyethyl-methacrylate, hydroxypropyl-methacrylate, hydroxyethylhexyl-methacrylate, and also optionally
(a6) of at least one cross-linking monomer or of at least one monomer comprising at least two olefinic unsaturations.

5. The method according to any of claims 1 to 4, wherein the mixture (M):
• also comprises a base, preferably an inorganic base, in particular a base chosen among NaOH, KOH, ammonium derivatives, ammonia, amine bases, for example triethanolamine, aminomethyl propanol, or 2-amino-2-methyl-propanol (AMP) and combinations thereof or
• has a pH greater than 5, preferably greater than 5.5, more preferentially greater than 6 or
• has a pH of less than 12 or
• has a pH ranging from 5 to 12, preferably from 5.5 to 12, more preferentially from 6 to 12 or
• does not comprise any surface-active compound or comprises a small amount, for example an amount of surface-active compound ranging from 0.05 to 10% by weight or ranging from 0.05 to 5% by weight, of the weight of the mixture (M) or an amount of non-ionic surface-active compound ranging from 0.05 to 10% by weight or ranging from 0.05 to 5% by weight, of the weight of the mixture (M).

6. The method according to any of claims 1 to 5, wherein the mixture (M) comprises from 0.5 to 15% by weight, preferably from 1 to 15% by weight or from 2 to 12% by weight, of rheology modifier.

7. The method according to any of claims 1 to 6, wherein:
• the preparation temperature is lower than the boiling point of the hydrophilic phase and lower than the boiling point of the lipophilic phase or
• the preparation temperature is higher than the melting point of the hydrophilic phase and higher than the melting point of the lipophilic phase or
• the preparation temperature is lower than the boiling point of the hydrophilic phase and lower than the boiling point of the lipophilic phase while being higher than the melting point of the hydrophilic phase and higher than the melting point of the lipophilic phase.

8. The method according to any of claims 1 to 7, wherein the dispersion is an emulsion of the dispersed lipophilic phase in the continuous hydrophilic phase.

9. The method according to any of claims 1 to 8, wherein the shear stress or the extensional stress:
• ranges from 300 to 5,000 Pa, preferably from 100 to 2,000 Pa or from 300 to 2,000 Pa, more preferentially from 100 to 1,700 Pa or from 300 to 1,700 Pa or
• is also applied when preparing the mixture (M), preferably at a value equal to or less than that applied when adding the lipophilic compound or
• the stress is applied using a device that produces a shear gradient of less than 2,000 s⁻¹ or less than 1,000 s⁻¹ or using a device that produces a shear gradient ranging from 100 to 5,000 s⁻¹ or ranging from 100 to 2,000 s⁻¹ or ranging from 100 to 1,000 s⁻¹, preferably ranging from 200 to 5,000 s⁻¹ or from 200 to 2,000 s⁻¹ or from 200 to 1,000 s⁻¹, in particular ranging from 200 to 800 s⁻¹.

10. The method according to any of claims 1 to 9, wherein the mean size (measured by light scattering) of the particles of dispersed lipophilic phase is submicronic, preferably at least 30% in volume or at least 40% in volume, preferably at least 50% in volume, the particles of dispersed lipophilic phase have a mean size that is nanometric or submicronic, more preferably at least 30% in volume or at least 40% in volume, more preferentially at least 50% in volume, the particles of dispersed lipophilic phase have a mean size ranging from 50 to 999 nm or from 100 to 999 nm, or from 50 to 990 nm or from 500 to 990 nm.

11. The method according to any of claims 1 to 10, also comprising:
• neutralisation of the dispersion (D), preferably by means of at least one compound chosen among NaOH, KOH, ammonium derivatives, ammonia, amine bases, for example triethanolamine, aminomethyl propanol or 2-amino-2-methyl-propanol (AMP) and combinations thereof or
• partial coacervation of the rheology-modifying compound, preferably:
∘ by reducing the pH of the dispersion (D), for example by reducing the pH to a value of less than 6.5, in particular by means of an acid compound, in particular by means of at least one organic or inorganic acid compound, in particular an acid compound chosen among phosphoric acid, citric acid, glucono-lactone, lactic acid, salicylic acid, glycolic acid, ascorbic acid, glutamic acid, hydrochloric acid, acetic acid, D-gluconic acid, sulphonic acid, methanesulphonic acid, benzimidazole sulphonic acid, tartaric acid, 4-aminobenzoic acid, benzoic acid, sorbic acid, phenylbenzimidazole sulphonic acid, benzylidene camphor sulphonic acid, terephthalylidene dicamphor sulphonic acid or
∘ by increasing the ionic strength of the dispersion (D), for example by adding at least one ionised compound or at least one salt, particularly NaCl, KCl, MgCl₂, CaCl₂,MgSO₄, CaSO₄ or
∘ by reducing the solubility of the rheology-modifying compound in the hydrophilic phase, for example by adding at least one cationic polymer, particularly by adding at least one cationic polymer, in particular a cationic polymer chosen among polyquaternium 1 to polyquaternium 47 and quaternised guars, in particular guar hydroxypropyltrimonium chloride, polydiallyldimethylammonium chloride (polyDADMAC or polyDDA), poly-2-(methacryloyloxy)ethyl-trimethylammonium chloride (polyMAD quat).

12. A dispersion (D) that can be prepared according to any of claims 1 to 11.

13. A product comprising at least one dispersion according to claim 12.

14. A use of a dispersion (D) according to claim 12 or of a product according to claim 13 in a field chosen among cosmetics, paints, dyes, printing, inks, construction, fuels, lubricants, anti-foaming agents, metallurgy, fertilisers, pharmaceuticals, agro-chemicals, crop protection products, detergents, food, leather, coating, in particular textile coating.
